# EUROPEAN PATENT APPLICATION

(11) **EP 4 796 132 A1**
(43) Date of publication of application: **26.08.2026**
(21) Application number: 25159470.1
(22) Date of filing: 21.02.2025
(51) Int. Cl.: A61L 9/012, A61L 9/014, A61L 9/015, A61L 9/02, A47L 11/00

(54) **A SCENTED STEAM APPLIANCE COMPRISING A REMOVABLE FRAGRANCE DISPENSING ELEMENT**

(71) Applicant: Laurastar SA, 1618 Châtel-St-Denis (CH)
(72) Inventor: DENISART, Jean-Luc, 1096 Cully (CH)
(74) Representative: Perreaud, Jérémie

(57) **Abstract**

The present invention concerns a scented steam appliance, comprising a base with a steam generator and a chamber having a hollow interior space. The chamber is fluidically connected to the steam generator via a first steam pathway to receive steam. A removable fragrance dispensing element is arranged inside the hollow interior space to come into contact with the steam, the fragrance dispensing element comprising a fragrance composition for scenting the steam. The appliance further includes a removable lid with a steam outlet, configured to close the chamber and direct the steam toward the steam outlet, allowing the scented steam to be expelled outside the appliance. The invention also concerns a removable fragrance dispensing element.

## Description

### FIELD OF THE INVENTION

The present invention concerns a steam appliance or a steam generator device, for example for domestic uses. In particular the present invention concerns a scented steam appliance or a scented steam generator device comprising a removable fragrance dispensing element. The invention also concerns a removable fragrance dispensing element for a scented steam appliance.

### BACKGROUND

In recent years, there has been a significant increase in the use of steam for a variety of tasks households. Steam is widely recognized for its versatility, being employed for cleaning, humidifying, ironing, and even for therapeutic and cosmetic purposes. Its ability to sanitize, moisturize, or soften surfaces makes it an attractive solution. Additionally, steam has become increasingly valued for its ability to carry fragrances, adding a sensory enhancement to its functional benefits.

Perfumed or scented steam is particularly appealing for applications where a pleasant scent can enhance the user experience. For example, scented steam can create a relaxing atmosphere during wellness treatments, improve the perceived cleanliness of a space or an object, especially when the object is cleaned or disinfected using scented steam, or infuse fabrics with a refreshing fragrance during ironing. However, despite these benefits, existing devices for producing scented steam often exhibit significant drawbacks in their design and operation.

A common issue with prior art devices is their arrangement, which can limit the effectiveness of the scenting process. Many devices fail to ensure that the fragrance is consistently or optimally diffused into the steam. This can result in a weak or uneven scenting effect, which diminishes the overall user experience. Furthermore, the components responsible for releasing the fragrance-such as scenting capsules or fragrance-dispensing elements-are often complex in structure or difficult for users to handle. In many cases, these elements require precise positioning or manipulation, leading to frustration and potential errors during installation. Users may find it challenging to ensure that the fragrance element is inserted correctly, which can impair the device's functionality.

In addition to functional challenges, there are concerns about the ecological impact of existing scenting elements. Prior art system utilizes disposable fragrance capsules or components that are not environmentally friendly. These components often contain synthetic materials or designs that contribute to unnecessary waste.

There are therefore needs for improved scented steam system or appliance to overcome the above drawback of the systems of the prior art.

### SUMMARY OF INVENTION

The present invention addresses these drawbacks by proposing a scented steam appliance according to claim 1 and a removable fragrance dispensing element according to claim 12. Other advantageous features can be found in the dependent claims.

The present invention concerns a scented steam appliance, and a removable fragrance optimized for steam scenting, user convenience, operational efficiency, and/or environmental sustainability.

The invention provides a simplified and optimized arrangement for the integration of fragrance dispensing element, ensuring consistent and effective scenting of the steam.

Furthermore, it introduces a more user-friendly system for replacing or maintaining the fragrance dispensing element, eliminating the need for complex manipulation.

Importantly, the invention emphasizes the use of ecological and sustainable materials, reducing waste and promoting responsible consumption.

By overcoming the limitations of prior art devices, this invention provides a significant advancement in the field of scented steam appliances, delivering a superior user experience while addressing environmental concerns.

The above and other objects, features, and advantages of the present invention and the manner of realizing them will become more apparent, and the invention itself will best be understood from a study of the following description with reference to the attached drawings showing some preferred embodiments of the invention.

### A BRIEF DESCRIPTION OF THE SEVERAL VIEWS OF THE DRAWINGS

Figure 1 shows a cross-sectional section of an embodiment of the scented steam appliance according to the invention in an operative position without the removable fragrance dispensing element arranged in the chamber;
Figure 2 depicts a cross-sectional section of an embodiment of the scented steam appliance according to the invention in an open position with the scented steam appliance, the removable fragrance dispensing element and the removable lid attached to an end piece being disassembled;
Figure 3 shows a cross-sectional section of an embodiment of the scented steam appliance according to the invention in the operative position with the removable fragrance dispensing element arranged in the chamber;
Figure 4 depicts another cross-sectional section of an embodiment of the scented steam appliance according to the disclosure in an operative position without the removable fragrance dispensing element arranged in the chamber
Figure 5 shows another cross-sectional section of an embodiment of the scented steam appliance according to the disclosure in an open position with the scented steam appliance, the removable fragrance dispensing element and the removable lid attached to an end piece being disassembled;
Figure 6 depicts another cross-sectional section of an embodiment of the scented steam appliance according to the disclosure in the operative position with the removable fragrance dispensing element arranged in the chamber; and
Figure 7 shows an embodiment of the removable fragrance dispensing element according to the disclosure.

Herein, identical reference numerals are used, where possible, to designate identical elements that are common to the Figures.

### DETAILED DESCRIPTION OF SEVERAL EMBODIMENTS

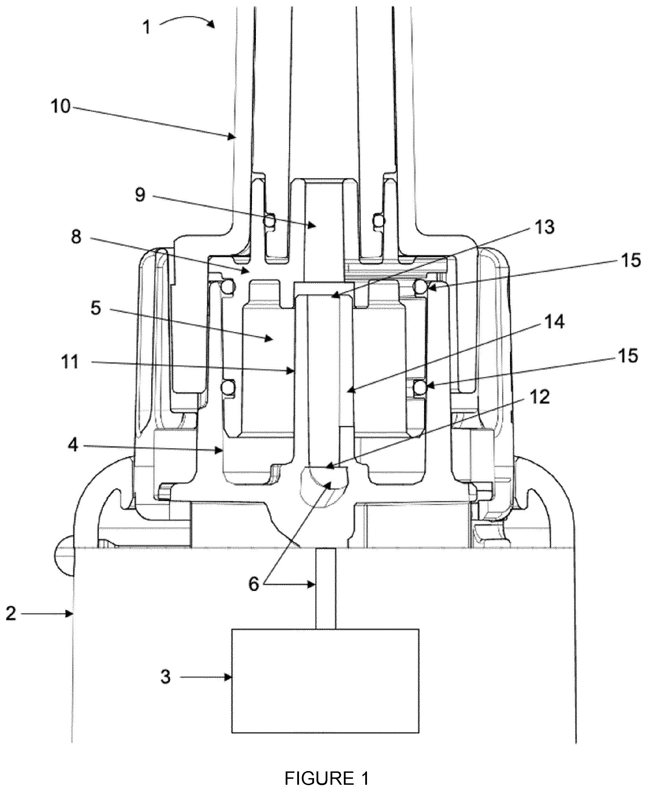

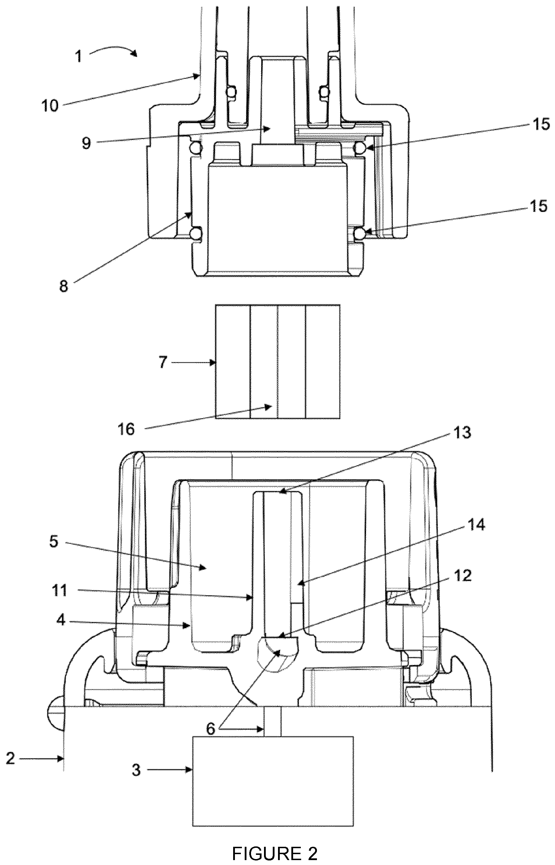

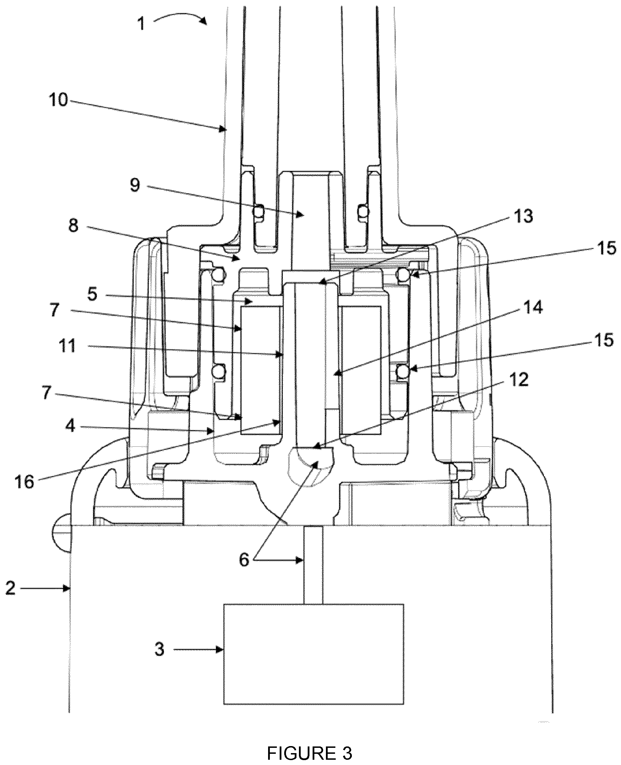

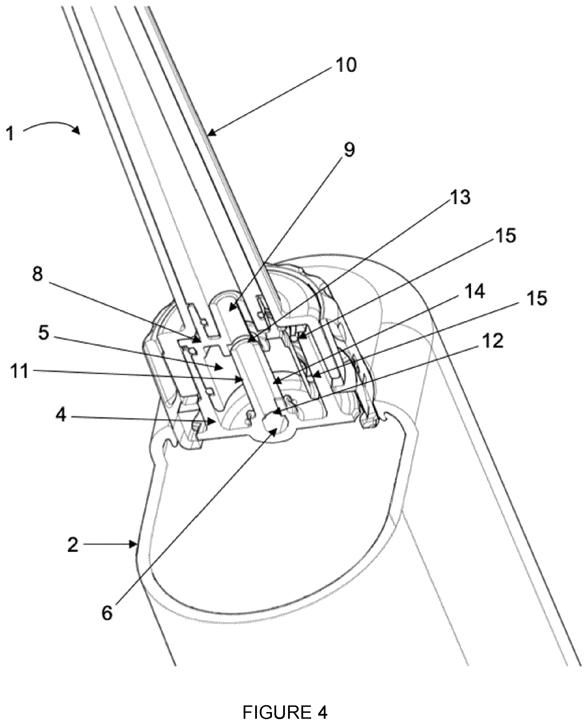

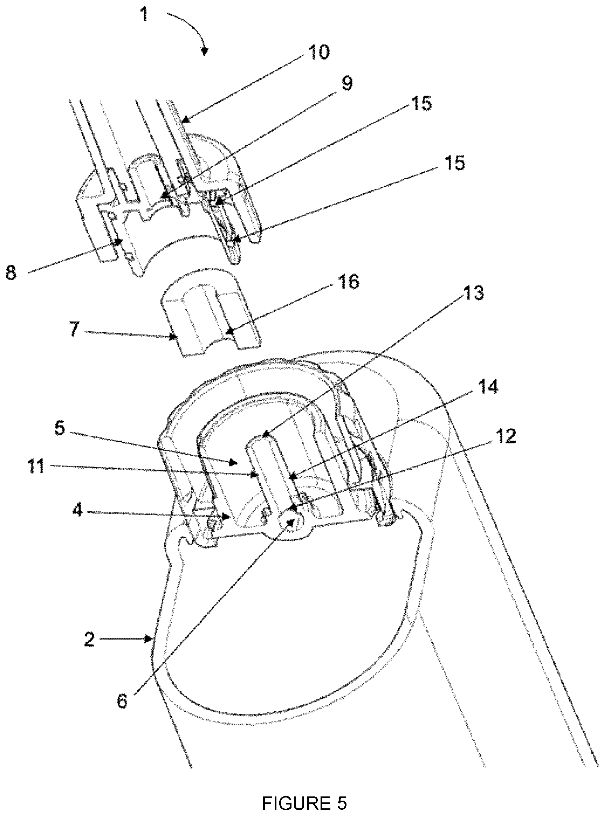

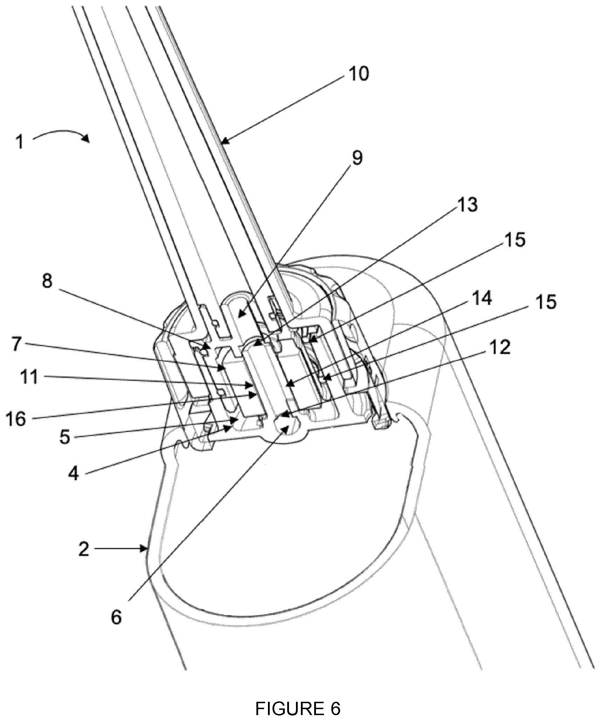

An exemplary scented steam appliance 1 or steam generator device according to the present disclosure is shown in figures 1 to 6.

The scented steam appliance 1 comprises a base 2 comprising a steam generator 3. The steam generator 3 is configured to produce steam from a fluid, such as water, supplied to it. The steam generator 3 may comprise a heating element or any other suitable means for converting the fluid into steam. Preferentially, the steam generator 3 is operatively connected to a power source, which may be integrated within the base 2 or connected externally, ensuring consistent and efficient operation. Additionally, the steam generator 3 may comprise control means, such as a thermostat, Pressure-sensitive control system or pressurestat, to regulate the temperature and/or pressure of the steam produced.

The scented steam appliance 1 comprises a chamber 4, preferentially arranged on the base 2, the chamber 4 comprising a hollow interior space 5 receiving steam from the steam generator 3 via a first steam pathway 6 fluidically connecting the steam generator 3 to the chamber 4.

The first steam pathway 6 is configured to fluidically connect the steam generator 3 to the chamber 4, allowing the steam produced by the steam generator 3 to be conveyed into the hollow interior space 5 of the chamber 4.

The first steam pathway 6 may comprise a conduit, channel, or similar means for guiding the steam, and is designed to ensure minimal heat and pressure loss during steam transfer. The first steam pathway 6 may further comprise sealing elements or thermal insulation to prevent steam leakage and maintain operational efficiency.

The connection between the steam generator 3 and the first steam pathway 6 or the first steam pathway 6 and the chamber 4 may be fixed or detachable, providing flexibility for maintenance or cleaning of the appliance

The chamber 4 comprises a hollow interior space 5 designed to receive and for example temporarily retain steam conveyed from the steam generator 3 via the first steam pathway 6.

The geometry of the chamber 4 may be cylindrical, rectangular, or any other suitable shape, depending on the intended application, to optimize steam distribution within the chamber.

The hollow interior space 5 enables the steam to circulate uniformly, ensuring consistent interaction with any elements housed within the chamber.

The chamber 4 may further comprise one or more internal surfaces that are smooth or textured to enhance steam flow or prevent condensation buildup.

The dimensions of the chamber 4 are selected to accommodate the required steam volume, maintaining sufficient capacity for efficient operation without causing pressure buildup. The chamber 4 may also comprise sealing features at its junctions to ensure that it is steam-tight during use.

The chamber 4 or its hollow interior space 5 is dimensioned to accommodate a removable fragrance dispensing element 7 which is positioned within its hollow interior space 5.

The chamber 4 comprises an opening configured to provide access to the hollow interior space 5. This opening allows for the insertion and removal of the removable fragrance dispensing element 7, facilitating maintenance and replacement.

Preferentially, the opening is positioned at the top of the chamber 4, opposite the base 2, enabling convenient user access without the need to disassemble other parts of the appliance. The placement of the opening at the top of the chamber 4 ensures ergonomic ease and aligns with the operational efficiency of the scented steam appliance.

The opening is dimensioned to allow easy handling of the fragrance dispensing element 7 while ensuring a secure fit to maintain the steam-tight integrity of the chamber 4 during operation.

The scented steam appliance further comprises a removable lid 8 to be arranged on the chamber 4 and to close the opening of the chamber 4.

The removable lid comprises a steam outlet 9 that allows the steam to be expelled or to flow outside the chamber 4 or outside the scented steam appliance 1.

The removable lid 8 is configured to close the chamber 4 and to direct the steam from the chamber to the steam outlet 9.

The removable lid 8 may comprises sealing means 15 configured to sealingly close the chamber 4 to avoid any steam leakage from the chamber 4 when the latter is closed by the lid 8.

The sealing means 15, such as gaskets or O-rings, may be provided on or around the removable lid 8 to ensure a steam-tight closure of the opening when the lid is in place. These sealing elements are configured to compress against the chamber 4 or its surrounding surfaces, creating a barrier that prevents steam from escaping. The gaskets or O-rings may be made of heat-resistant and durable materials, such as silicone or rubber, to withstand the high temperatures and pressures associated with steam generation. This arrangement ensures that the appliance operates efficiently by maintaining consistent steam pressure and preventing energy loss or unintended release of steam.

Alternatively, the sealing means 15 may be arranged on surfaces of the chamber 4.

The removable lid 8 may be connected to an end piece 10 or be integral or be an end piece 10. Therefore, with this arrangement, the chamber 4 and the removable fragrance dispensing element 7 are positioned at the end of the overall steam flow path originating from the steam generator 3. This configuration ensures that the chamber 4 serves as the final point of steam flow before the steam exits the appliance 1 via the steam outlet 9. By situating the chamber 4 at the end of the steam path, the design optimizes the interaction between the steam and the fragrance dispensing element 7 housed within the chamber 4, as the steam remains concentrated and directed. The interaction also between the steam and other parts of the appliance along the flow path is minimized, thereby preserving the intensity and quality of the scented steam.

This arrangement also facilitates the replacement of the removable dispensing element 7 as the latter is located within the chamber 4 at the terminal end of the steam flow path, accessible through the removable lid 8. This positioning allows the user to easily access the dispensing element 7 without the need to disassemble other components of the appliance. The accessibility provided by this design ensures quick and straightforward replacement or maintenance, thereby improving user convenience and reducing downtime during operation.

The end piece 10 may also be interchangeable and take the form of different accessories, allowing the scented steam appliance 1 to adapt to various applications and user needs. For example, the end piece 10 may be configured as a diffuser for evenly dispersing scented steam into a room, a nozzle for directing steam onto specific surfaces or fabrics, or an attachment designed for therapeutic or cosmetic uses, such as facial steaming. These interchangeable end pieces 10 can be easily connected to or detached from the appliance, providing versatility while ensuring user convenience.

In certain embodiments, the end piece 10 may include specific functional features, such as adjustable vents or perforations to control the steam flow rate and direction. Additionally, some end pieces may incorporate integrated elements, such as brushes or scrapers, for use in cleaning or fabric care applications. The end piece 10 may be made of materials resistant to heat and moisture, such as high-grade plastics or metals, ensuring durability and consistent performance during operation.

This modular design not only enhances the functionality of the appliance but also offers users the flexibility to customize the appliance for specific tasks. Furthermore, the interchangeability of the end piece 10 facilitates easy maintenance and replacement, extending the lifespan of the appliance and improving overall user satisfaction.

In an embodiment, the scented steam appliance 1 may comprise a central hollow shaft 11 extending inside the chamber 4.

The central hollow shaft comprises one end 12 connected to the first steam pathway 6 and a second end 13 connected to the steam outlet 9, when the removable lid 8 closes or is arranged on the chamber 4.

The central hollow shaft 11 comprise at least one opening 14, arranged on the side of the shaft 11 and being an opening in the lateral wall of the shaft. The at least one opening 14 may be elongated along the central hollow shaft 11, on at least a portion of the latter.

The at least one opening 14 enable the steam being in the hollow space of the hollow shaft 11 to enter the chamber 4 or flow into the chamber 4 and vice-versa.

The inclusion of the central hollow shaft 11 provides a structured pathway for directing steam within the chamber 4, enhancing the efficiency of steam flow and fragrance diffusion. By connecting one end or the first end 12 of the hollow shaft 11 to the first steam pathway 6 and the other end or second end 13 to the steam outlet 9, the hollow shaft ensures that steam is properly guided through the chamber 4 during operation. The at least one opening 14, arranged on the lateral wall of the hollow shaft 11, allows steam to flow between the hollow interior of the shaft and the chamber 4, enabling uniform circulation of steam within the chamber. This design promotes optimal interaction between the steam and the fragrance dispensing element 7, ensuring consistent scenting of the steam.

The openings 14, which may be elongated along the central hollow shaft 11, further improve steam distribution by allowing controlled flow into and out of the chamber 4. This configuration minimizes turbulence within the chamber and ensures even exposure of the fragrance dispensing element 7 to the steam.

Additionally, the central hollow shaft 11, which may take the form of a hollow tube made of metal or other suitable materials, contributes to the structural integrity of the appliance while withstanding high temperatures and pressures generated by the steam.

By integrating the central hollow shaft 11 with openings 14, the appliance achieves a balance between efficient steam flow and effective fragrance diffusion, enhancing the performance and reliability of the scented steam appliance. This arrangement also simplifies maintenance by ensuring that the removable fragrance dispensing element 7 is easily accessible, while maintaining consistent functionality of the steam pathway.

During the operation of the scented steam applicant 1, steam generated by the steam generator 3 is conveyed into the chamber 4 through the first steam pathway 6, which is fluidly connected to the chamber 4 or the hollow interior space 5. Upon entering the chamber 4, the steam comes into contact with the removable fragrance dispensing element 7, which is positioned within the hollow interior space 5. As the steam interacts with the fragrance dispensing element 7, it absorbs the fragrance composition contained within or released by the element, resulting in the production of scented steam. The scented steam is then directed toward the steam outlet 9, which is integrated into the removable lid 8 that closes the opening of the chamber 4. The steam outlet 9 ensures that the scented steam is efficiently expelled from the appliance, providing consistent and uniform diffusion of the fragrance.

In an embodiment, the chamber 4 is arranged in a remote module operatively connected to the base 2, which comprises the steam generator 3. The operative connection between the base 2 and the remote module may include a fluid connection, such as a conduit, tubing, or flexible hose, allowing the steam produced by the steam generator 3 to be conveyed directly to the chamber 4.

This configuration allows for physical separation between the steam generation process and the chamber 4, providing greater flexibility in the appliance's design and use. The remote module housing the chamber 4 is fluidly connected to the base 2 via a conduit or tubing, enabling steam produced by the steam generator 3 to be efficiently conveyed to the chamber 4. This arrangement minimizes heat transfer to the base 2 and other sensitive components, enhancing safety and energy efficiency. Additionally, the modular design facilitates portability, allowing the remote module to be placed in various locations or orientations, depending on the intended application. This setup also simplifies maintenance and replacement of components, such as the fragrance dispensing element 7, by allowing easy access to the chamber 4 without interfering with the base 2.

The steam appliance may include an electrical connection or a battery to power its various components and functionalities. The electrical connection may be configured to supply power to the steam generator, control circuits, or other operational elements of the appliance through a standard power cord or adapter. Alternatively, the appliance may be equipped with a rechargeable or replaceable battery, allowing for cordless operation and increased portability.

The steam appliance may further comprise an on/off button configured to control the activation of the steam generator. This button allows the user to easily turn the appliance on to begin steam production or off to cease operation, providing simple and intuitive control. The on/off button may be positioned on the base 2 of the appliance for easy accessibility, and may include an indicator, such as an light, to signal the operational status of the appliance. In certain embodiments, the on/off button may also be integrated with safety features, such as a delayed start or automatic shut-off mechanism, to enhance user safety and prevent overheating.

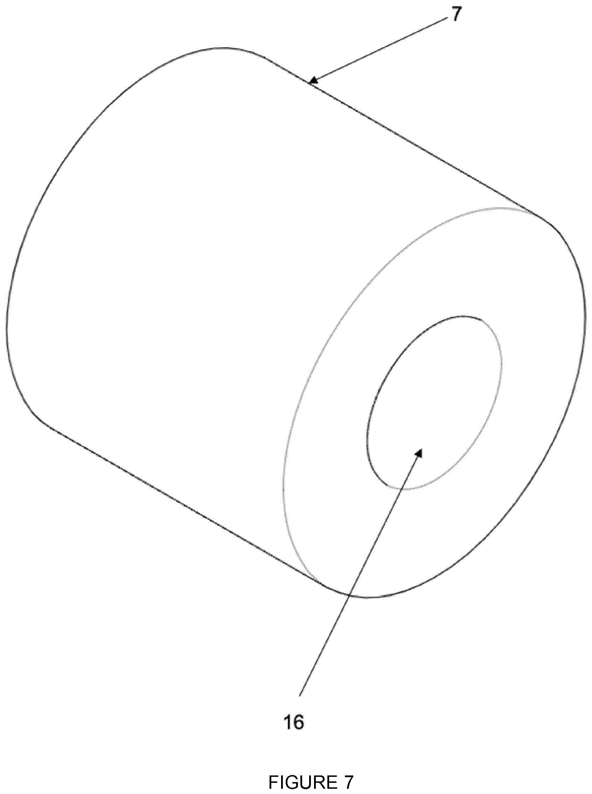

An exemplary removable fragrance dispensing element 7 according to the present disclosure is shown in figure 7.

The removable fragrance dispensing element 7 is intended to be arranged in a scented steam appliance according to the present disclosure.

The removable fragrance dispensing element 7 comprises or be integrally made of a first material comprising a fragrance composition configured to release a fragrance or a scent or a perfume or an aroma into the steam as the steam passes through or over the fragrance dispensing element 7.

The fragrance dispensing element 7 may comprise, be infused or be impregnated with the fragrance composition.

Preferentially, the fragrance dispensing element 7 is a solid element or in the solid form or may comprise porous material which may act as a substrate or a carrier material comprising or being impregned with the fragrance composition.

The removable fragrance dispensing element 7 may also comprise or be integrally made of wood. The wood may comprise or be impregnated with the fragrance composition.

The wood serves as a natural substrate for absorbing and releasing the fragrance composition when exposed to steam. The wood may be untreated or treated to enhance its fragrance-absorbing properties and impregnated with a fragrance composition suitable for release upon contact with steam. The use of wood provides an environmentally friendly alternative to synthetic materials and aligns with sustainability objectives. Various types of wood, such as cedar, sandalwood, or other aromatic species, may be used to enhance the olfactory appeal of the scented steam.

The fragrance composition may be in the form of a liquid, gel, or solid and can include essential oils, synthetic fragrances, or a combination of both.

The fragrance composition may include a wide variety of oils and essential oils known for their aromatic and therapeutic properties. Essential oils, such as lavender, eucalyptus, peppermint, citrus, or sandalwood, are particularly advantageous due to their natural origins and well-documented benefits, including relaxation, invigoration, and stress relief. These oils can be impregnated into the fragrance dispensing element 7 to ensure consistent release of the aroma into the steam.

In addition to essential oils, the fragrance composition may comprise fragrance oils, which are synthetic or blended aromatic compounds. Fragrance oils allow for a broader range of scents and are particularly useful in applications requiring custom or unique fragrance profiles. These oils may be formulated to mimic natural aromas or create entirely novel scent experiences.

The fragrance composition may further include alcohol-based solutions, which are particularly effective for ensuring rapid evaporation and diffusion of the fragrance into the steam. Alcohol-based solutions can carry both synthetic and natural fragrances, providing flexibility in the formulation of the scent.

Water-based fragrance solutions are also suitable for use in the fragrance dispensing element 7 including natural fragrances derived from flowers, herbs, or fruits, offering a sustainable and environmentally friendly alternative.

The fragrance composition may be selected from a list comprising oil, essential oil, fragrance oil, alcohol-based solution, or water-based fragrance solution, and may also include combinations of these.

For example, an essential oil may be blended with an alcohol-based solution to enhance diffusion, or a fragrance oil may be mixed with a water-based solution to create a balanced scenting composition. Such combinations provide versatility and customization, allowing the fragrance dispensing element 7 to meet a wide range of user preferences and functional requirements.

In certain embodiments, the fragrance composition may also include additives to enhance its properties, such as stabilizers, dispersants, or encapsulation agents. These additives ensure that the fragrance is released consistently and efficiently when the fragrance dispensing element 7 interacts with the steam.

In another embodiment, the removable fragrance dispensing element 7 has a cylindrical shape or is cylindrical. The cylindrical geometry is particularly advantageous for optimizing steam flow, as it ensures that the steam can interact uniformly with the surface of the fragrance dispensing element. The cylindrical shape also facilitates the secure placement of the element within the hollow interior space 5 of the chamber 4, while allowing for easy insertion and removal. This configuration enhances the efficiency of the scenting process and ensures compatibility with different chamber designs.

In a further embodiment, the removable fragrance dispensing element 7 comprises a central hole 16 dimensioned to accommodate the central hollow shaft 11 when the fragrance dispensing element is arranged inside the chamber 4. The central hole 16 may traversing the removable fragrance dispensing element 7.

The central hole 16 ensures proper alignment of the fragrance dispensing element and allows the central hollow shaft 11 to direct steam through or around the element. This arrangement simplifies the installation process, ensuring that the fragrance dispensing element is correctly positioned during operation and easily removable when needed.

The removable fragrance dispensing element 7 may positioned at least partially above the at least one opening 14 when arranged inside the chamber 4. This arrangement ensures that the steam exiting the opening 14 from the central hollow shaft 11 comes into direct contact with the fragrance dispensing element 7. The positioning allows the steam to pass through or over the fragrance dispensing element 7, optimizing the absorption and diffusion of the fragrance composition into the steam. This configuration enhances the efficiency of the scenting process by ensuring maximum interaction between the steam and the fragrance dispensing element 7 while maintaining consistent steam flow within the chamber 4. Additionally, the placement of the fragrance dispensing element 7 above the opening 14 minimizes turbulence and promotes uniform diffusion of the scented steam throughout the appliance.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention not to be limited to the described embodiments and be given the broadest reasonable interpretation in accordance with the language of the appended claims. The features of any one of the above-described embodiments may be included in any other embodiment described herein.

## Claims

1. A scented steam appliance (1) comprising:
a base (2)
a steam generator (3);
a chamber (4) comprising a hollow interior space (5) receiving steam from the steam generator via a first steam pathway (6) fluidically connecting the steam generator (3) and the chamber (4);
a removable fragrance dispensing element (7) arranged inside the hollow interior space (5) such that the removable fragrance dispensing element (7) is in contact with the steam, the removable fragrance dispensing element (7) comprising a fragrance composition and;
a removable lid (8) comprising a steam outlet (9) that allows the steam to be expelled outside of the scented steam appliance (1), the removable lid (8) being configured to close the chamber (4) to direct the steam from the chamber (4) to the steam outlet (9).

2. The scented steam appliance (1) according to claim 1, wherein the chamber (4) comprises a central hollow shaft (11) extending inside the chamber (4), the central hollow shaft (11) comprising:
one end (12) connected to the first steam pathway (6);
a second end (13) connected to the steam outlet (9), when the removable lid (8) closes the chamber (4); and
at least one lateral opening (14) configured to allow steam to flow from the central hollow shaft (11) into the hollow interior space (5).

3. The scented steam appliance (1) according to claim 1 or 2, wherein the removable lid comprises sealing means (15) configured to sealingly close the chamber.

4. The scented steam appliance (1) according to the preceding claims, wherein the removable lid is connected to an end piece (10) or is integral of an end piece (10).

5. The scented steam appliance (1) according to the preceding claims, wherein the removable fragrance dispensing element (7) is a solid element.

6. The scented steam appliance (1) according to the preceding claim, wherein the removable fragrance dispensing element (7) comprises wood.

7. The scented steam appliance (1) according to the preceding claims, wherein the removable fragrance dispensing element (7) has a cylindrical shape.

8. The scented steam appliance (1) according to claim 5, 6 and 7 , wherein the removable fragrance dispensing element (7) comprises a central hole (16) dimensioned to accommodate the central hollow shaft (11) when the removable fragrance dispensing element (7) is arranged inside the chamber (4).

9. The scented steam appliance (1) according to claims 2 to 9 , wherein when the removable fragrance dispensing element (7) is arranged at least partially above the at least one opening (14) when arranged inside the chamber (7).

10. The scented steam appliance (1) according to the preceding claims, wherein the fragrance composition is selected from the list comprising oil, essential oil, fragrance oil, alcohol-based solution or water-based fragrance solution.

11. The scented steam appliance (1) according to the preceding claims, wherein the chamber (4) is arranged in a remote module operatively connected to the base (2) comprising the steam generator (3).

12. A removable fragrance dispensing element (7) for a scented steam appliance, comprising a first material comprising a fragrance composition, wherein the removable fragrance dispensing element has a cylindrical shape and comprises a central hole traversing the removable fragrance dispensing element.

13. The removable fragrance dispensing element (7) according to claim 12, wherein the first material is infused with the fragrance composition.

14. The removable fragrance dispensing element (7) according to claim 12 or 13, wherein the first material comprises wood.

15. The removable fragrance dispensing element (7) according to claims 12 to 14, wherein fragrance composition is selected from the list comprising oil, essential oil, fragrance oil, alcohol-based solution or water-based fragrance solution.
